(19) **Europäisches Patentamt / European Patent Office / Office européen des brevets**

(11) **EP 3 394 290 B1**

(12) **EUROPEAN PATENT SPECIFICATION**

(45) Date of publication and mention
of the grant of the patent:
**29.09.2021 Bulletin 2021/39**

(21) Application number: **16829076.5**

(22) Date of filing: **22.12.2016**

(51) Int Cl.:
***C12Q 1/68*** *(2018.01)*

(86) International application number:
**PCT/EP2016/082411**

(87) International publication number:
**WO 2017/109089 (29.06.2017 Gazette 2017/26)**

(54) **DIFFERENTIAL DIAGNOSIS IN GLIOBLASTOMA MULTIFORME**

DIFFERENTIALDIAGNOSE BEI GLIOBLASTOMA MULTIFORME

DIAGNOSTIC DIFFÉRENTIEL EN CAS DE GLIOBLASTOME MULTIFORME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2015 EP 15202328**

(43) Date of publication of application:
**31.10.2018 Bulletin 2018/44**

(73) Proprietor: **Helmholtz Zentrum München - Deutsches
Forschungszentrum für Gesundheit und Umwelt (GmbH)
85764 Neuherberg (DE)**

(72) Inventors:
• **BELKA, Claus**
**80337 München (DE)**
• **NIYAZI, Karim-Maximilian**
**81377 München (DE)**
• **UNGER, Kristian**
**85622 Feldkirchen (DE)**

• **ZITZELSBERGER, Horst**
**85764 Oberschleißheim (DE)**
• **PITEA, Adriana**
**La Jolla, California 92093-0688 (US)**
• **MITTELBRONN, Michel**
**79100 Freiburg (DE)**

(74) Representative: **Schiweck Weinzierl Koch
Patentanwälte Partnerschaft mbB
Ganghoferstraße 68 B
80339 München (DE)**

(56) References cited:
**WO-A2-2012/089753      WO-A2-2015/004413**

• **SUJAYA SRINIVASAN ET AL: "A Ten-microRNA Expression Signature Predicts Survival in Glioblastoma", PLOS ONE, vol. 6, no. 3, 1 January 2011 (2011-01-01) , page e17438, XP055055815, ISSN: 1932-6203, DOI: 10.1371/journal.pone.0017438**

EP 3 394 290 B1

SEQUENCE LISTING

<110> Helmholtz Zentrum München

<120> Differential diagnosis in glioblastoma multiforme

<130> Y1686 PCT

<150> EP 15 20 2328.9<151> 2015-12-23

<160> 4

<170> BiSSAP 1.3

<210> 1
<211> 22
<212> DNA
<213> Homo sapiens

<220>
<223> hsa-miR-615-5p

<400> 1
gggggucccc ggugcucgga uc          22

<210> 2
<211> 24
<212> DNA
<213> Homo sapiens

<220>
<223> hsa-miR-125a-5p

<400> 2
ucccugagac ccuuuaaccu guga          24

<210> 3
<211> 22
<212> DNA
<213> Homo sapiens

<220>
<223> hsa-let-7a-5p

<400> 3
ugagguagua gguuguauag uu          22

<210> 4
<211> 22
<212> DNA
<213> Homo sapiens

<220>
<223> hsa-let-7b-5p

<400> 4
ugagguagua gguugugugg uu          22

**Description**

The present invention relates to a kit consisting of means for detecting and/or quantifying all four of the following miRNAs: hsa-miR-615-5p; hsa-miR-125a-5p; hsa-let-7a-5p; and hsa-let-7b-5p, wherein said means consist of probes or primers, which are specific for the respective miRNAs, for use in a method of stratifying GBM or a method of diagnosing higher-risk GBM or lower-risk GBM as described herein.

Malignant gliomas account for approximately 70% of primary brain tumors diagnosed in adults. Median age at diagnosis is 64 years with men being more frequently affected than women (Fisher et al., Neurol. Clin. 2007; 25:867-90, vii.). Amongst all gliomas, glioblastoma multiforme (GBM) is the most common and aggressive form (Louis et al., Acta Neuropathol. 2007; 114:97-109). State-of-the-art treatment of GBM comprises surgical resection and adjuvant radiochemotherapy followed by maintenance chemotherapy. Implementation of temozolomide (TMZ) into the radiochemotherapeutic regime improved 2-year survival rates of patients with newly diagnosed malignant glioma (mainly GBM) from 11% to 27%, 3-year survival rates from 4% to 16%, and 5-year survival rates from 2% to 10% (Stupp et al., Lancet. Oncol. 2009; 10:459-66; Stupp et al., N. Engl. J. Med. 2005; 352:987-96). Recent prospective phase III trials (AVAglio & RTOG) evaluated the efficacy of TMZ-based radiochemotherapy in combination with the anti-angiogenic agent bevacizumab as first-line therapy. Unfortunately, these trials failed to show improvements in overall survival, but significant and marginally significant benefits in progression-free survival were observed (Chinot et al., N. Engl. J. Med. 2014; 370(8):709-722; Gilbert et al., N. Engl. J. Med. 2014; 370:699-708). Integrin targeting by cilengitide also failed to provide an additional benefit over classical TMZ-based radiochemotherapy according to the EORTC/NCIC26981/22981-NCIC CE3 trial (Stupp et al., Lancet. Oncol. 2014; 15:1100-8). Thus, the results of molecularly targeted treatment approaches so far confirm TMZ-based radiochemotherapy as standard treatment for GBM.

The advent of high precision treatment planning algorithms, image-guided dose application, and novel irradiation qualities, as well as the introduction of TMZ have provided clear therapeutic improvements for primary and recurrent GBM (Niyazi et al., Radiat Oncol. 2014; 9:299; Niyazi et al., Radiat Oncol. 2013; 8:287; Niyazi et al., Radiat. Oncol. 2011; 6:177). However, prognosis of most GBM patients still remains dismal with a high rate of local recurrence, emphasizing the clear need for further optimization (Niyazi et al., Radiat. Oncol. 2014; 9:299; Weber et al., Radiother Oncol. 2009; 93:586-92). At present, several strategies are being followed in this regard: Firstly, more elaborate imaging techniques as well as improved image-guidance during radiotherapy are being tested (Cyran et al., Radiat. Oncol. 2014; 9:3; Gilbert et al. RTOG 0625: A phase II study of bevacizumab with irinotecan in recurrent glioblastoma (GBM), 2009; Niyazi et al., Radiother Oncol. 2011; 98:1-14; Stall et al., Radiat. Oncol. 2010; 5:5.). Secondly, various molecularly designed substances are undergoing pre-clinical and clinical testing for their therapeutic efficacy in combination with radio(chemo)therapy (Beal et al., Radiat. Oncol. 2011; 6:2; Flieger et al., J. Neurooncol. 2014; 117:337-45; Niyazi et al., Int. J. Radiat. Oncol. Biol. Phys. 2010; Stupp et al., J. Clin. Oncol. 2010; 28:2712-8; Welsh et al., Radiat Oncol. 2009; 4:69.). These targeted treatment approaches require molecular stratification of patients in order to identify the subgroups that can benefit most from a given strategy. Classical radiochemotherapy also displays wide inter-individual differences in terms of response and survival rates (Frenel et al., Bulletin Du Cancer. 2009; 96:357-367). Accordingly, numerous efforts are undertaken in order to characterize the molecular mechanisms orchestrating therapy sensitivity and resistance and to identify prognostic and predictive markers.

So far, only few prognostic factors have been defined for GBM, including age and Eastern Cooperative Oncology Group (ECOG) score. In addition, involvement of the subventricular zone and extent of resection are known to be of negative prognostic values (Adeberg et al., Radiation Oncology (London, England). 2014; 9:95). More recently, the first molecular markers have been established. In this regard, methylation of the O6-methylguanine DNA-methyltransferase (MGMT) promoter region was recognized to be of positive predictive value for the efficacy of TMZ-based radiochemotherapy, and molecular profiling of long-term survivors disclosed the positive prognostic value of a proneural-like expression pattern linked to mutations in the genes encoding for isocitrate dehydrogenases 1/2 (IDH1/2) (Hegi et al., J. Clin. Oncol. 2008; 26:4189-99; Kim Hak Jae et al., Radiation oncology (London, England), 2012; 7:39; Reifenberger et al., Int. J. Cancer. 2014; 135:1822-31).

WO 2012/089753 relates to a method for diagnosing and/or prognosing of glioblastoma based on the determination of expression profiles of sets of miRNAs representative for glioblastoma compared to a reference. Furthermore, WO 2012/089753 relates to sets of polynucleotides and/or primer pairs for detecting sets of miRNAs for diagnosing and/or prognosing of glioblastoma in a biological sample from a subject. Further, WO 2012/089753 relates to means for diagnosing and/or prognosing of glioblastoma comprising said sets of primer pairs and/or polynucleotides. In addition, WO 2012/089753 relates to a kit for diagnosing and/or prognosing of glioblastoma comprising means for determining expression profiles of sets of miRNAs representative for glioblastoma and at least one reference. Further, WO 2012/089753 relates to the use of polynucleotides and/or primer pairs for diagnosing and/or prognosing of glioblastoma in a biological sample of a subject.

Srinivasan et al. ("A ten-microRNA expression signature predicts survival in glioblastoma", PLoS ONE, 2011, vol. 6, issue 3) describes a microRNA (miRNA) expression signature that can predict GBM patient survival. The miRNA expression data of GBM patients (n = 222) derived from a dataset were analyzed. 10 significant miRNAs using Cox regression analysis on the training set were identified and a risk score based on the expression signature of these miRNAs was formulated that segregated the patients into high and low risk groups with significantly different survival times.

During the last years, microRNAs (miRNAs) have increasingly received attention. With a high degree of promiscuity miRNAs target and regulate several mRNA species encoding for proteins involved in various signaling pathways (Ha Minju, Kim V. Narry, Nature Reviews Molecular Cell Biology, 2014; 15:509-524). Accumulating evidence indicates that miRNA expression signatures can serve as biomarkers for diagnosis and risk assessment of diverse malignancies, including GBM (Cheng Wen et al., Oncotarget. 2015.; De Smaele et al., Brain Res. 2010; 1338:100-11; Gao et al., Oncogene, 2014; 0; Jiang et al., American Journal of Pathology, 2010; 177:29-38; Manterola et al., Neuro-Oncology, 2014: not218; Sana et al., Carcinogenesis, 2014; 35:2756-2762; Shou et al., Experimental and Therapeutic Medicine, 2015; 9:167-171; Zhang et al., PLoS ONE, 2014; 9:e96908). Given that the available prognostic markers can segregate GBM patients only to a limited extent, additional and better markers and/or signatures are sought for. Moreover, in many instances the prior art studies did not select GBM patients, which receive the standard-of-care treatment, which limits their usefulness.

The technical problem underlying the present invention can be seen in the provision of alternative or improved methods for stratifying GBM patients. This problem is solved by the subject-matter of the claims.

Accordingly, the present invention, in a first aspect, relates to a kit consisting of means for detecting and/or quantifying all four of the following miRNAs: hsa-miR-615-5p; hsa-miR-125a-5p; hsa-let-7a-5p; and hsa-let-7b-5p, wherein said means consist of probes or primers, which are specific for the respective miRNAs, for use in a method of stratifying GBM or a method of diagnosing higher-risk GBM or lower-risk GBM as described herein.

The term "microRNA", abbreviated "miRNA", has its art-established meaning. It designates small RNAs, which are encoded by the genome of an animal or the human genome. MiRNAs do not encode proteins or peptides. The understanding of their function is still emerging. Generally speaking, they frequently play a role in gene regulation including gene silencing. The size of miRNAs is between a few dozen and a few hundred nucleotides. In terms of structure, miRNAs in many instances are hairpin molecules, i.e. they consist of a single strand which falls back upon itself, thereby giving rise to a stem and a loop, wherein within the stem there may be bulges and/or non-paired nucleotides. MiRNAs are typically the result of the processing of pre-miRNAs, which are larger in size and more complex in structure (several loops and several stems). A recent review of the field of miRNAs is Minju & Narry, *loc. cit.*

The four miRNAs, namely hsa-miR-615-5p; hsa-miR-125a-5p; hsa-let-7a-5p; and hsa-let-7b-5p, are the mature forms of the corresponding precursors. The nomenclature given conforms with that published by the mirBase consortium (Griffiths-Jones S, Grocock RJ, van Dongen S, Bateman A, Enright AJ, Nucl. Acids Res, 2006, 34:D140-D144). In more detail, "hsa" refers to the species (i.e. homo sapiens) of origin of the sequence, the capital letter "R" indicates the mature form of the respective miRNAs, and -5p and -3p, respectively, defines from which part of the precursor stem-loop the miRNA is derived from.

The sequences of the four miRNAs are set forth by SEQ ID NOs: 1 to 4.

As will be apparent from the enclosed examples, the present inventors surprisingly found that a very small set of miRNAs, namely four miRNAs, may be used to stratify GBM patients in a highly reliable manner. While already each single miRNA is able to statistically significantly predict overall survival of GBM patients, the set of the four miRNAs as defined above is used herein.

The kit in accordance with the first aspect contains means for detecting and/or quantifying all four of the miRNAs in accordance with the present invention.

It is further described herein that said kit comprises no means for detecting and/or quantifying miRNAs or RNAs other than all four of hsa-miR-615-5p; hsa-miR-125a-5p; hsa-let-7a-5p; and hsa-let-7b-5p, wherein said kit optionally comprises means for quantifying one or more standard RNAs.

This clarifies that the open language ("comprising") means the presence of further subject-matter such as vessels, carriers, or manuals, but does not extend to the provision of entire arrays, which are designed for assessing expression levels of more than four, such as hundreds of miRNAs. It is further described herein that the kit allows for the presence of means for detecting and/or quantifying RNAs other than the four miRNAs according to the invention, this is confined to means for quantifying one or more standard RNAs. It is further described herein that that standard RNAs in turn serve as an internal standard, which aids in quantifying the expression levels of the four miRNAs according to the invention. RNAs, which qualify as internal standards are generally those, which are not regulated differentially. It is further described herein that while preference is given to those standard RNAs, which are not miRNAs, it is not totally excluded to use also one or more miRNAs as standards, especially to the extent they are not differentially regulated.

Since miRNA regulation is highly dynamic, in general they do not qualify as standards to be used in miRNA expression normalization. For normalization constitutively expressed small non-coding RNAs such as RNU6, SNORD61, SNORD65 and/or SNORD95 may be used. These small RNAs are larger in size (45-200 nt) compared to miRNAs (usual range: 21-23 nt) and have been shown to exhibit constant expression. Further is described herein the use of SNORD61 for normalization.

Normalization is generally effected by dividing the expression data of the miRNA to be normalized by the expression data of a constitutively expressed RNA.

It is further described herein that the kit for use of the present invention may comprise a manual, which manual comprises instructions for performing methods of the present invention disclosed further below.

Means for detecting and/or quantifying are disclosed further below and consist of probes or primers specific for the miRNAs of the present invention.

In a second aspect, the present invention provides a method of stratifying glioblastoma multiforme (GBM), said method comprising or consisting of determining for a GBM patient all four of the expression levels of miRNAs hsa-miR-615-5p, hsa-miR-125a-5p, hsa-let-7a-5p and hsa-let-7b-5p, wherein (a) the more the expression level of hsa-miR-615-5p is increased and the more the expression levels of hsa-miR-125a-5p, hsa-let-7a-5p and hsa-let-7b-5p are decreased, the worse is the prognosis of said patient; (b) determining whether an expression level is increased or decreased is by comparison with a reference state, said reference state being the average expression levels in GBM of hsa-miR-615-5p, hsa-miR-125a-5p, hsa-let-7a-5p and hsa-let-7b-5p, respectively; and (c) diagnostic methods practised on the human or animal body as well as methods for treatment of the human or animal body by surgery are excluded.

The term "stratifying" has its art-established meaning, especially in the sense as it is used in the field of diagnostics and pharmacogenomics. Accordingly, "stratifying GBM" relates to a differential diagnosis of GBM, which permits to identify two or more groups or a continuum with regard to a given parameter within GBM patients. From a pharmacogenomics perspective, said stratifying serves to identify subgroups within all GBM patients, which subgroups respond favourably to treatment. As will become apparent below, in the present case the diagnostic and the pharmacogenomic aspect of stratifying GBM generally coincide.

Stratifying allows the assignment of GBM patients to two or more groups or ordering them with regard to a given parameter. I.e., stratifying does not necessarily imply the binning of patients. Instead, a given property of patients may scale with the deviation of the expression levels of the miRNAs from the reference state, i.e. the larger the deviation from the reference state, the larger will be the expected value for the given parameter. In accordance with the second aspect of the invention, said parameter is the prognosis for a given GBM patient. The more the expression levels are increased and decreased in accordance with item (a) of the second aspect, the worse is the prognosis of the patient.

The term "prognosis" has its art-established meaning in the field of medicine. In particular, it designates the probable outcome of a patient with regard to measures such as recurrence, overall survival, cancer specific survival and others. In the context of glioblastoma multiforme usually the outcome of interest is overall survival. The median overall survival of glioblastoma multiforme patients is about 14 months, however, overall survival varies very much on the individual level. "Worse prognosis" in accordance with the present invention accordingly defines preferably an expected survival time of the GBM patient under consideration of less than about 14 months. "Average expression levels in GBM" as recited in item (b) of the second aspect accordingly are expression levels, which are indicative of an expected survival time of about 14 months. Patients are treated according to standard of care (SOC; see Stupp et al., loc. cit.).

As noted above, the reference state is defined in terms of the average expression levels in glioblastoma multiforme of the four miRNAs of the invention. Ideally, said average expression levels are determined using as many GBM patients as possible. This would be in favor of unbiased sampling. In practical terms, said average expression levels may also be determined for smaller cohorts of GBM patients. For example, if the method of the invention is to be practised at a given site, such as a hospital, said reference state may be defined in terms of the average expression levels for all GBM patients, which are treated in said hospital and gave consent to determining the expression levels of the four miRNAs according to the invention.

The following correlation between diagnostic and pharmacogenomic stratification applies. The worse the prognosis of a patient, the less likely it is that any of the above described therapies of GBM has a beneficial effect. If no beneficial effects are to be expected, and giving due consideration to side effects of the given treatment, it may be adequate to dispense with certain forms of treatment altogether. Such a decision, however, can only be made if simple reliable and rapid means of determining the prognosis for a given patient are available. Such means are provided by the present invention.

The determination of expression levels can be done by any suitable method. Such methods include, but are not confined to quantitative PCR for which the primers as disclosed below may be employed.

Alternatively, expression profiling can be done using miRNA arrays. miRNA arrays may be manufactured for the purpose of the present invention in a tailored manner in which case they would contain probes for the four of the miRNAs of the invention and optionally probes for one or more standards. Standards are preferably non-differentially regulated RNAs as disclosed herein above. Alternatively, commercially available miRNA arrays may be used. CBC of Heidelberg, Germany, is a manufacturer of miRNA arrays. In accordance with the present invention, it is preferred that the expression data obtained with such arrays (which may comprise probe sets for more than four miRNAs) are used to the extent they relate to said four miRNAs used in the present invention and optionally one or more standard RNAs.

The terms "increased" and "decreased", respectively, preferably relate to statistically significant increased and statistically significant decreased expression levels of the miRNAs. Related to the second aspect, the present invention provides a method of stratifying glioblastoma multiforme (GBM), said method comprising or consisting of determining for a GBM patient all four of the expression levels of miRNAs hsa-miR-615-5p, hsa-miR-125a-5p, hsa-let-7a-5p and hsa-let-7b-5p, wherein (a) the more the expression level of hsa-miR-615-5p is increased and the more the expression levels of hsa-miR-125a-5p, hsa-let-7a-5p and hsa-let-7b-5p are decreased, the worse is the prognosis of said patient; (b) determining whether an expression level is increased or decreased is by comparison with a reference state, said reference state being the average expression levels in GBM of hsa-miR-615-5p, hsa-miR-125a-5p, hsa-let-7a-5p and hsa-let-7b-5p, respectively, and diagnostic methods practised on the human or animal body as well as methods for treatment of the human or animal body by surgery are excluded.

In a preferred embodiment of the method according to the second aspect of the present invention, (i) an expression level of hsa-miR-615-5p, which is increased and expression levels of hsa-miR-125a-5p, hsa-let-7a-5p and hsa-let-7b-5p, which are decreased are indicative of bad prognosis; and (ii) an expression level of hsa-miR-615-5p, which is decreased, and expression levels of hsa-miR-125a-5p, hsa-let-7a-5p and hsa-let-7b-5p, which are increased, are indicative of good prognosis.

The term "good prognosis" designates a life-expectancy, which is above the average life-expectancy of GBM patients. The term "bad prognosis" designates a life-expectancy, which is below the average life-expectancy. The average life-expectancy is determined across all GBM patients. As noted above, where possible, as many patients as available are used for determining the average. In a particular embodiment, the average is determined for a random sample of at least 50, at least 100, at least 200, at least 500 or at least 1,000 GBM patients. These patients may be recruited worldwide and/or may be of Caucasian and/or Japanese origin. Such definitions of "average" apply throughout this specification.

The terms "good prognosis" and "lower-risk GBM" are used equivalently herein. Similarly, the terms "bad prognosis" and "higher-risk GBM" are also used equivalently in this specification.

In a further preferred embodiment of the method of the second aspect, said method does not comprise assessing expression levels of miRNAs or RNAs other than all four of hsa-miR-615-5p; hsa-miR-125a-5p; hsa-let-7a-5p; and hsa-let-7b-5p, wherein said method optionally comprises quantifying the expression level(s) of one or more standard RNAs.

It is a key feature of the invention that a surprisingly small set of four miRNAs provides an excellent predictor of the prognosis of GBM patients. Accordingly, to the extent expression levels of further RNAs than the four specific miRNAs of the invention are to be quantified, this is confined to standard RNAs.

In a third aspect, the present invention provides a method of diagnosing higher-risk GBM or lower-risk GBM, said method comprising or consisting of subjecting a sample taken from a GMB patient to a method for determining expression levels and determining a risk score R for the GBM patient, R being defined as follows: $R = \sum_i a_i f_i$ wherein i runs from 1 to 4; $a_1$ is between 0.30 and 0.40, and is preferably 0.33; $a_2$ is between -0.35 and -0.25, and is preferably -0.28; $a_3$ is between 0.45 and 0.55, and is preferably 0.51; and $a_4$ is between -1.00 and -0.90, and is preferably -0.97; and $f_1$ to $f_4$ are the expression levels of miRNAs hsa-miR-615-5p, hsa-miR-125a-5p, hsa-let-7a-5p and hsa-let-7b-5p; wherein an average risk score $R_{av}$ is defined as the average of R in GBM; wherein a value of $(R - R_{av})$, which is greater than zero, is indicative of higher-risk GBM, and a value of $(R - R_{av})$, which is smaller than zero, is indicative of lower-risk GBM; and wherein diagnostic methods practised on the human or animal body as well as methods for treatment of the human or animal body by surgery are excluded.

The risk score R is a weighted sum of the expression levels $f_1$ to $f_4$ of the four miRNAs according to the invention. While this is not a requirement, it is preferred to scale the expression data prior to calculating the risk score. The scaling step renders the expression levels determined by different methods of measurement comparable. A standard scaling procedure was used which moves the mean value distribution towards 0 by subtracting a so-called Windsorized mean (i.e. mean of all values, but the most extreme values, preferably the 30% lowest and 30% highest values, are excluded) and scaling the bandwidth of the value distribution by division with the standard deviation.

Any of the art-established methods for determining expression levels may be used. These include, but are not confined to microarray-based assays, quantification using RNA-sequencing, and qRT-PCR.

These and other methods are capable of generating (semi-)quantitative expression values of the miRNAs and standard non-coding RNAs.

Expression levels are preferably determined from tissue samples. Said tissue samples may be fresh, fresh-frozen or formalin-fixed paraffin-embedded (FFPE) tissue sections.

Alternatively, it is also envisaged to use of body fluids, especially blood, serum or plasma.

For the average of R, the same considerations apply as for the above mentioned reference state and the average expression levels.

Preferred intervals for $a_1$ are between 0.30 and 0.35 and between 0.32 and 0.34. Preferred intervals for $a_2$ are between -0.30 and -0.25 and between -0.29 and -0.27. Preferred intervals for $a_3$ are between 0.50 and 0.55 and between 0.50 and 0.52. Preferred intervals for $a_4$ are between -1.0 and -0.95 and between -0.98 and -0.96.

Related to the third aspect, the present invention provides a method of diagnosing higher-risk GBM or lower-risk GBM, said method comprising or consisting of subjecting a sample taken from a GMB patient to a method for determining expression levels and determining a risk score R for the GBM patient, R being defined as follows: $R = \sum_i a_i$

$f_i$, wherein i runs from 1 to 4; $a_1$ is between 0.30 and 0.40, and is preferably 0.33; $a_2$ is between -0.35 and -0.25, and is preferably -0.28; $a_3$ is between 0.45 and 0.55, and is preferably 0.51; and $a_4$ is between -1.00 and -0.90, and is preferably -0.97; and $f_1$ to $f_4$ are the expression levels of miRNAs hsa-miR-615-5p, hsa-miR-125a-5p, hsa-let-7a-5p and hsa-let-7b-5p; wherein an average risk score $R_{av}$ is defined as the average of R in GBM; wherein a value of (R - $R_{av}$), which is greater than zero, is indicative of higher-risk GBM, and a value of (R - $R_{av}$), which is smaller than zero, is indicative of lower-risk GBM, wherein diagnostic methods practised on the human or animal body as well as methods for treatment of the human or animal body by surgery are excluded.

In a preferred embodiment of the methods according to the second and third aspect of the invention, said method comprises subjecting a sample taken from the GBM patient to an analytical method, said analytical method employing the means as defined in accordance with the first aspect.

The analytical methods are the methods for determining expression levels as disclosed herein.

In a preferred embodiment of the methods according to the second and third aspect of the invention, said GBM patient is isocitrate dehydrogenase 1 (IDH1) mutation negative.

In a low proportion of glioblastoma multiforme (GBM) patients (about 5%) there is a mutation of the isocitrate dehydrogenase 1 (IDH1) gene; see Reuss et al. (Acta Neuropathol. (2015); 129:133-146). A typical mutation in the human IDH1 protein is R132H. Generally speaking, an IDH1 mutation is a typical feature of lower grade (less than WHO grade IV) gliomas. As regards glioblastomas (grade IV) with an IDH1 mutation, it is generally considered the tumors were initially of lower grade and have acquired the mutation in the course of tumorigenesis. Typically, GBM with an IDH1 mutation exhibits better prognosis. On the other hand, GBM with no mutation in the IDH1 gene generally has bad prognosis. As a consequence, there is a particular interest in analyzing, diagnosing and stratifying those GBM patients, which do not have a mutation in the IDH1 gene.

Thus, the present invention provides a kit as defined herein for use in a method of stratifying GBM or a method of diagnosing higher-risk GBM or lower-risk GBM, said method being the method of the second or third aspect, respectively.

Related thereto, the present invention provides in a fourth aspect a use of the kit as defined in the first aspect for stratifying GBM or for diagnosing higher-risk GBM or lower-risk GBM, wherein diagnostic methods practised on the human or animal body as well as methods for treatment of the human or animal body by surgery are excluded, and wherein said diagnosing is effected by the method of the second or third aspect, respectively.

In preferred embodiments of the second, third and fourth aspect, said higher-risk GBM is not responsive to postoperative radiotherapy.

As noted above, higher-risk GBM, i.e. those forms of GBM, which are characterized by bad prognosis, include or consist of forms of GBM, which are not responsive to treatment, especially not to post-operative radiotherapy.

Higher-risk vs. lower-risk is also distinguishable in terms of hazard ratio. Hazard ratio is the instantaneous risk of dying relative to a baseline hazard. For the higher-risk group a hazard ratio of 3.79 was found in the discovery cohort, and 2.11 in the validation cohort, respectively. Here the baseline corresponds to the lower-risk group.

In preferred embodiments of the use according to the fourth aspect, said kit and/or said means, respectively, further comprise(s) a carrier.

The carrier may be solid carrier or a gel. The carrier may be such that it has a planar surface or it may take the shape of beads. Carriers may be used for the purpose of implementing the kit as mini-array. According to the first aspect of the present invention, implementations of the above disclosed means consist of probes or primers, which are specific for the respective miRNAs. Means and methods for attaching such means to the surface of a carrier are known in the art.

In further preferred embodiments of the use of the kit in accordance with the invention, said kit further comprises a manual describing the method of the second or third aspect, respectively.

For the kit for use or the use of the kit according to the present invention, said means consist of probes or primers, which are specific for the respective miRNA, wherein optionally said probes or primers are immobilized on the carrier as defined above.

Preferred lengths of said primers and probes, respectively, are between 15 and 50, preferably between 18 and 30 nucleotides.

Preferred specific forward primers are given in Table 1 below:

**Table 1:** Primers for detecting or quantifying miRNAs in accordance with the invention.

| miRNA | SEQ ID NO: | sequence |
| --- | --- | --- |
| hsa-miR-615-5p | 1 | 5'GGGGGUCCCCGGUGCUCGGAUC |
| hsa-miR-125a-5p | 2 | 5'UCCCUGAGACCCUUUAACCUGUGA |
| hsa-let-7a-5p | 3 | 5'UGAGGUAGUAGGUUGUAUAGUU |

(continued)

| miRNA | SEQ ID NO: | sequence |
|---|---|---|
| hsa-let-7b-5p | 4 | 5'UGAGGUAGUAGGUUGUGUGGUU |

In a further preferred embodiment of the kit for use or the use of the kit, said stratifying and said diagnosing is to be effected for GBM patients, which are IDH1 mutation negative.

In further preferred embodiments of the method in accordance with the second and the third aspect, said determining comprises the use of probes and/or primers, which are specific for the respective miRNA, said use preferably being for determining the expression level of the respective miRNAs.

It is further described herein: If not expressly indicated otherwise, preferred embodiments of one aspect of the invention apply *mutatis mutandis* also to other aspects of the invention, i.e. they define also preferred embodiments of said other aspects of the invention.

The figures show:

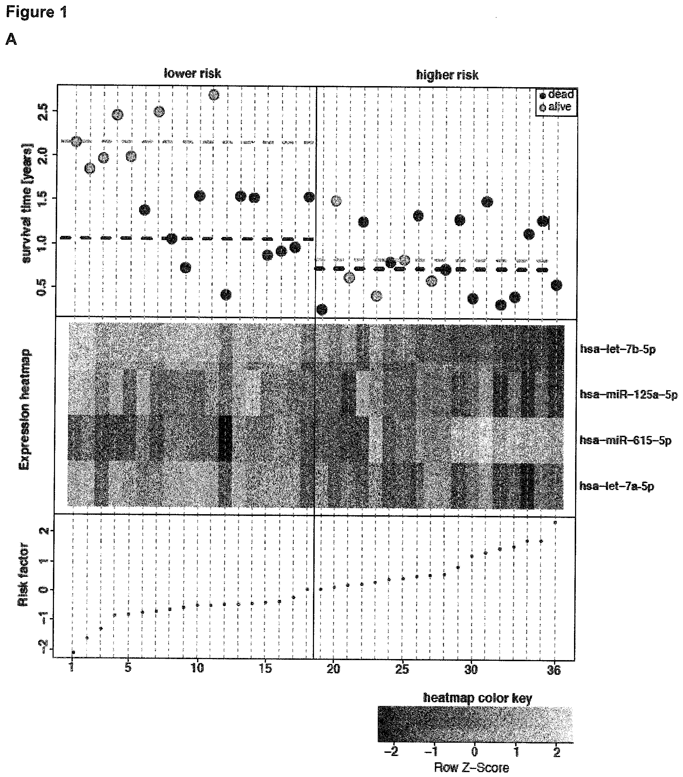

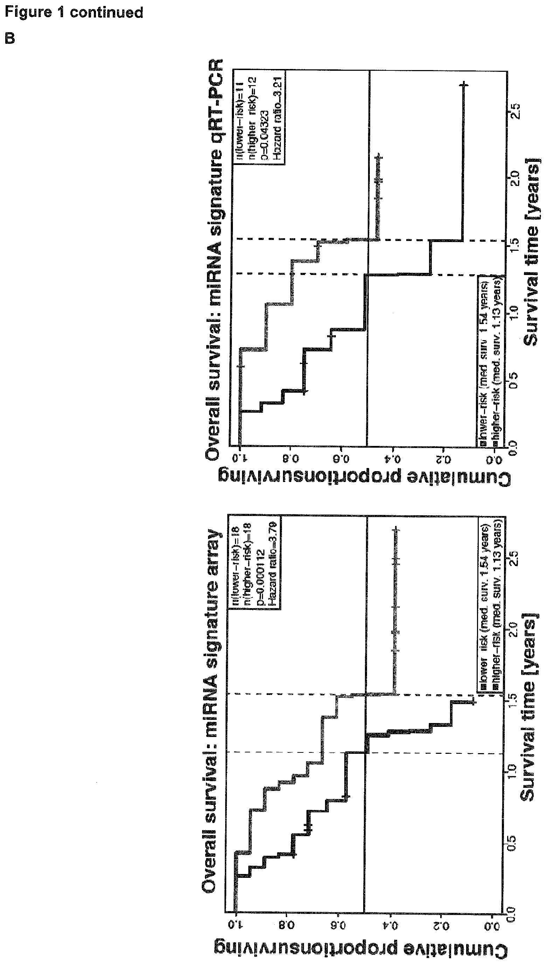

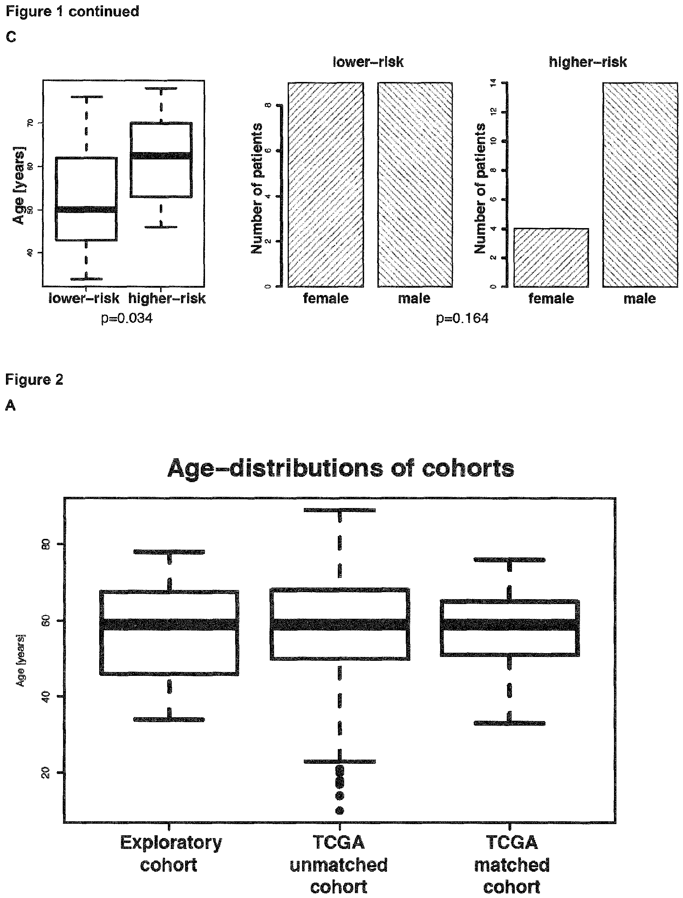

Figure 1. Extraction of a 4-miRNA signature as independent predictive marker for the overall survival of GBM patients in the exploratory cohort.

(A) Overall survival (top panel), hierarchical cluster heat map of miRNA array expression levels (middle panel), and risk factors calculated on the basis of miRNA expression values and Coxph coefficients (bottom panel) for all patients. The median risk factor value was used to classify higher-risk and lower-risk patients.

(B) Kaplan-Meier overall survival analyses of higher-risk and lower-risk GBM patients. Higher-risk and lower-risk patients were stratified based on the risk factors calculated from the Coxph coefficients and the miRNA expression levels as measured in the microarray (left panel, 35 patients) or by qRT-PCR analyses (right panel, 19 patients). Hazard ratios and p-values were calculated by log-rank test. (C) Distribution of age (left panel) and sex (middle and right panels) in higher-risk and lower-risk GBM patients. Statistical comparison was performed by Student's t-test and Fisher's exact, respectively.

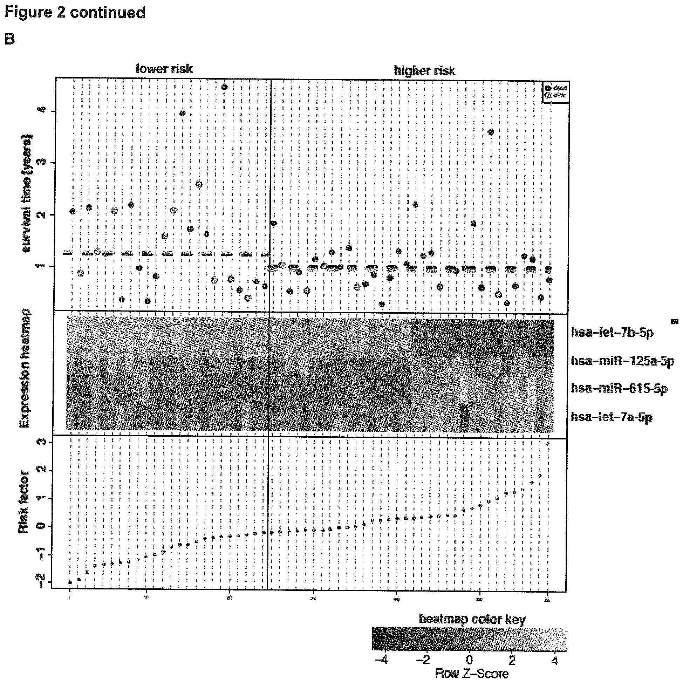

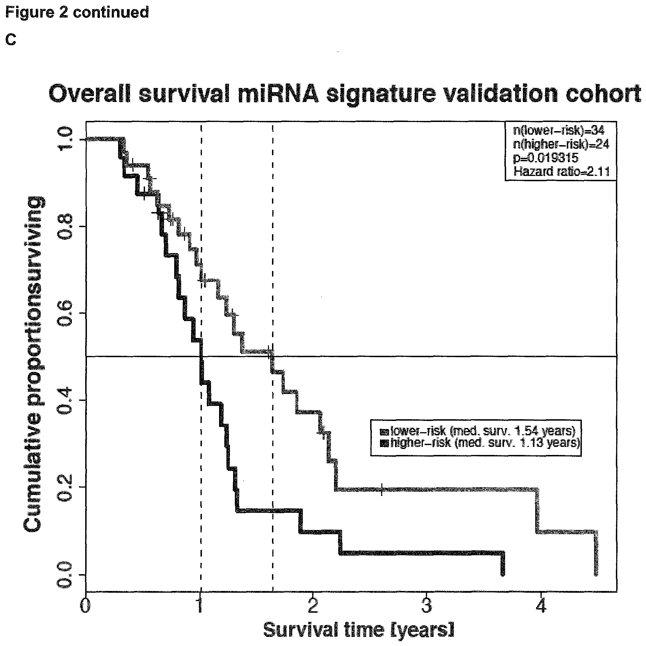

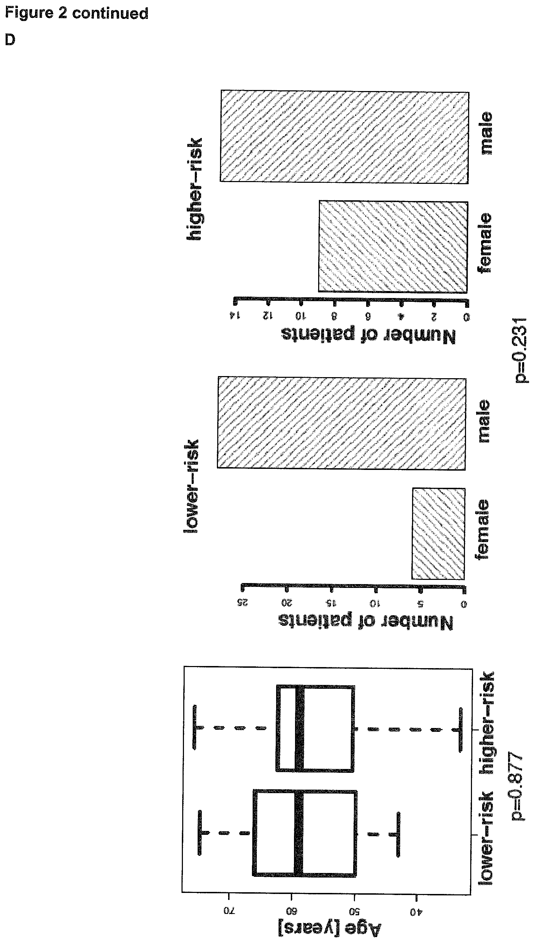

Figure 2. Evaluation of the prognostic value of the extracted 4-miRNA signature in an age- and sex-matched subgroup of the TCGA GBM dataset.

(A) Age distribution in the exploratory cohort and the TCGA GBM cohort before and after age matching. (B) Overall survival (top panel), hierarchical cluster heat map of miRNA expression levels (middle panel), and risk factors for patients of the age- and sex-matched TCGA GBM cohort. The median risk factor value was used to classify higher-risk and lower-risk patients.

(C) Kaplan-Meier overall survival analyses of higher-risk and lower-risk patients of the age- and sex-matched TCGA GBM cohort. Classification of higher-risk and lower-risk patients was performed on the basis of the risk factors calculated from the Coxph coefficients (Tab. 1) and the miRNA expression levels. Hazard ratios and p-values were calculated by log-rank test. (D) Distribution of age (left panel) and sex (middle and right panel) in higher-risk and lower-risk patients of the age- and sex-matched TCGA GBM cohort. Student's t-test and Fisher's exact test were employed for statistical comparisons.

Figure 3. Kaplan-Meier plot of high-risk (red) and low-risk (blue) patients defined using the 4-miRNA signature of the invention.

Figure 4. Kaplan-Meier plot of high-risk (red) and low-risk (blue) patients of the IDH1 mutation negative subgroup defined using the 4-miRNA signature of the invention.

The following examples illustrate the invention.

## Example 1

Low complexity miRNA signature and evaluation of its prognostic significance for overall survival

A signature that consisted of the four miRNAs hsa-let-7a-5p, hsa-let-7b-5p, hsa-miR-125a-5p and hsa-miR-615-5p, which was statistically significantly associated with overall survival (p-value = 0.0048), was found. The median risk score calculated from the expression levels of the signature miRNAs and the corresponding coxph coefficients (Table 2) separated the patients into a high- and a lower-risk group.

**Table 2**

**Table 2.** Cox-proportional hazard coefficients used in risk score calculation

| miRNA | coefficient |
|---|---|
| hsa-let-7b-5p | -0.9669152 |
| hsa-miR-125a-5p | -0.2821517 |
| hsa-miR-615-5p | 0.3254795 |
| hsa-let-7a-5p | 0.5059587 |

Cox regression analyses of the high- and the lower-risk groups revealed a 5.11 fold increased risk of death (95% CI: 2.03-12.85) for the higher-risk group compared to the lower-risk group (p-value = 0.000112). The median survival time was 13.5 months for patients of the higher-risk group and 18.4 months for patients of the lower-risk group, respectively. These results were visualized by Kaplan-Meier overall survival curves (Fig. 1B). Univariate testing of the individual miRNAs within the signature revealed p-values in the range between 0.0015 and 0.016, indicating that each single miRNA was able to statistically significantly predict overall survival. Fig. 1A summarizes the survival data of the patients in relation to the calculated risk scores and expression levels.

**Example 2**

Independent in silico validation of the detected miRNA signature

For the purpose of independent validation, the miRNA signature was tested in an age-matched miRNA data subset of an independent GBM cohort downloaded from the TCGA database (Cancer Genome Atlas Research Network. Comprehensive genomic characterization defines human glioblastoma genes and core pathways Nature, 2008; 455:1061-8). The high- and the lower-risk groups were defined by using the median risk score of the discovery set to dichotomize the patients of the validation set. The resulting coxph model revealed a hazard ratio of 2.11 (95% CI 1.13-3.91) and a p-value of 0.019 (Fig. 2D). Fig. 2B summarizes the survival data of the patients of the validation cohort in relation to calculated risk scores and expression levels.

**Example 3**

Technical validation of signature by qRT-PCR

In order to technically validate the 4-miRNA signature and to support potential applicability in clinical routine diagnostics, the inventors measured the expression of the four miRNAs in a subset of samples (n = 23), for which residual material was available by qRT-PCR. Analogous cox proportional hazard analysis with the qRT-PCR data confirmed the results obtained with the miRNA array data. Patients of the higher-risk group revealed significantly impaired overall survival (p-value = 0.043) and a hazard-ratio of 3.21 (95% CI 1.02-10.16) as compared to patients of the lower-risk group. (Fig. 1B).

**Example 4**

Validation in independent cohort

*Material and Methods*

Patients cohort

A retrospective cohort of 41 patients who were treated according to the Stupp et al. (Lancet Oncol (2009); 10:459-466) standard-of-care protocol was recruited from the LMU Neuropathology and Radiation Oncology Departments' registries. 17% of patients were younger and 83% were older than 50 years with a median age of 64.5 years. 46% of patients were male and 54% were female. Median follow-up time was 1.61 years (95% CI: 1.05-2.38). 5 out of the 41 patients were isocitrate dehydrogenase 1 (IDH1) mutation positive. With regard to MGMT promoter methylation status, 10 patients were MGMT-promoter methylation negative and 31 were positive.

RNA isolation

From the formalin-fixed paraffin-embedded tissue blocks that were prepared from resected tumours 5 micron HE reference slides were used to identify areas of tumour tissue with > 80% tumour cell content that were used for macrodissection of five serial 10 micron sections. The macrodissected tissue was subjected to RNA isolation after deparaffinisation as described in Niyazi et al. (Oncotarget (2016); 7:45764-45775).

Quantitative real-time PCR

Total RNA containing the small non-coding RNA fraction was subjected to SYBR green chemistry based qRT-PCR as described in Niyazi et al. (2016). The measured median CT values of the signature miRNAs were transformed to delta Cts by subtracting CTs of the reference small non-coding RNA SNORD68 per sample. The inverted values were used as expression values and the resulting expression matrix was scaled per miRNA.

Calculation of risk scores

Using the cox-proportional hazard coefficients from the initial signature model as published in the Niyazi et al. study, risk scores were calculated for each patient. The patients were assigned to the high- and low-risk group using the threshold published in the Niyazi et al. (2016) study, followed by Kaplan-Meier analysis for the calculation of the log-rank score statistics and hazard-ratio.

*Results*

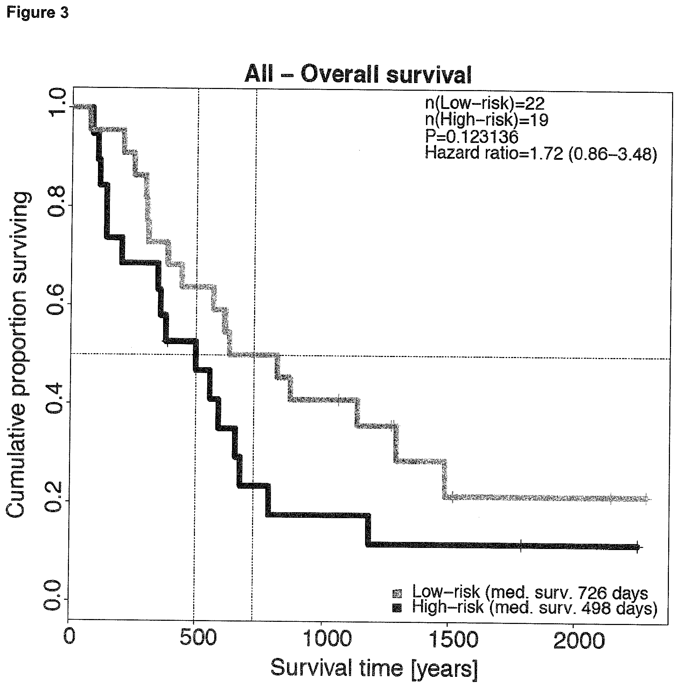

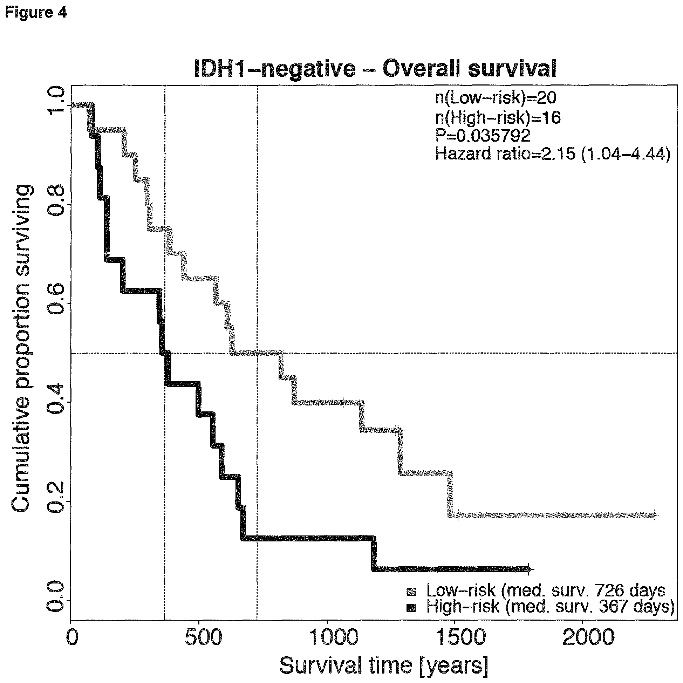

In the complete group of patients (n = 41), a trend of high-risk patients with worse survival compared to low-risk patients was obvious (Fig. 3). When excluding patients bearing an IDH1 mutation, the difference in overall survival between high-risk and low-risk patients is statistically significant (p-value: 0.036, hazard-ratio: 2.15 (95%-CI: 1.05-4.44), Fig. 4). Further, univariate and multivariate analysis showed that the signature risk score in the investigated cohort is independent of the risk factors age, sex, MGMT methylation.

*Discussion*

In the IDH1 mutation negative subgroup of patients the difference in overall survival between high-risk and low-risk patients was statistically significant. This is an important finding since IDH1 mutation positive GBM patients (secondary GBMs) are known to have a much better prognosis compared with IDH1 mutation negative ones (de novo GBMs). Thus, from a clinical perspective, IDH1 mutation negative GBM patients build the clinical relevant group for a personalised risk-stratified treatment. Moreover, from a pathological point of view IDH1 mutation positive GBM are considered being secondary GBMs emerging from lower-grade gliomas (Reuss (2015)). Therefore, IDH1 mutation positive tumours comprise a different etiology of gliomas, which is also reflected in the prognosis of affected patients (Cai et al., Oncoscience (2016); 3:258-265).

The inventors of the present invention were able to validate the 4-miRNA signature of the invention in the IDH1 mutation negative subgroup of a second independent cohort of patients. This finding substantiates the prognostic value of the signature and provides further evidence for its validity.

## Claims

1. A method of stratifying glioblastoma multiforme (GBM),
   said method comprising or consisting of determining for a GBM patient all four of the expression levels of miRNAs
   hsa-miR-615-5p, hsa-miR-125a-5p, hsa-let-7a-5p and hsa-let-7b-5p,
   wherein

(a) the more the expression level of hsa-miR-615-5p is increased and the more the expression levels of hsa-miR-125a-5p, hsa-let-7a-5p and hsa-let-7b-5p are decreased, the worse is the prognosis of said patient;
(b) determining whether an expression level is increased or decreased is by comparison with a reference state, said reference state being the average expression levels in GBM of hsa-miR-615-5p, hsa-miR-125a-5p, hsa-let-7a-5p and hsa-let-7b-5p, respectively; and
(c) diagnostic methods practised on the human or animal body as well as methods for treatment of the human or animal body by surgery are excluded.

2. The method of claim 1, wherein

(i) an expression level of hsa-miR-615-5p, which is increased, and expression levels of hsa-miR-125a-5p, hsa-let-7a-5p and hsa-let-7b-5p, which are decreased, are indicative of bad prognosis; and
(ii) an expression level of hsa-miR-615-5p, which is decreased, and expression levels of hsa-miR-125a-5p, hsa-let-7a-5p and hsa-let-7b-5p, which are increased, are indicative of good prognosis.

3. The method of claim 1 or 2, wherein said method does not comprise assessing expression levels of miRNAs or RNAs other than all four of hsa-miR-615-5p; hsa-miR-125a-5p; hsa-let-7a-5p; and hsa-let-7b-5p, wherein said method optionally comprises quantifying the expression level(s) of one or more standard RNAs.

4. A method of diagnosing higher-risk GBM or lower-risk GBM, said method comprising or consisting of subjecting a sample taken from a GMB patient to a method for determining expression levels and determining a risk score R for the GBM patient, R being defined as follows:

$$R = \Sigma_i\, a_i\, f_i$$

wherein

i runs from 1 to 4;
$a_1$ is between 0.30 and 0.40, and is preferably 0.33; $a_2$ is between -0.35 and -0.25, and is preferably -0.28; $a_3$ is between 0.45 and 0.55, and is preferably 0.51; and $a_4$ is between -1.00 and -0.90, and is preferably -0.97; and $f_1$ to $f_4$ are the expression levels of miRNAs hsa-miR-615-5p, hsa-miR-125a-5p, hsa-let-7a-5p and hsa-let-7b-5p;
wherein an average risk score $R_{av}$ is defined as the average of R in GBM;
wherein a value of ($R - R_{av}$), which is greater than zero, is indicative of higher-risk GBM, and a value of ($R - R_{av}$), which is smaller than zero, is indicative of lower-risk GBM; and
wherein diagnostic methods practised on the human or animal body as well as methods for treatment of the human or animal body by surgery are excluded.

5. The method of any one of claims 1 to 4, wherein said GBM patient is isocitrate dehydrogenase 1 (IDH1) mutation negative.

6. A kit consisting of means for detecting and/or quantifying all four of the following miRNAs:

hsa-miR-615-5p; hsa-miR-125a-5p; hsa-let-7a-5p; and hsa-let-7b-5p, wherein said means consist of probes or primers, which are specific for the respective miRNAs,
for use in a method of stratifying GBM or a method of diagnosing higher-risk GBM or lower-risk GBM, said method being the method of any one of claims 1 to 5.

7. Use of the kit of claim 6 for stratifying GBM or for diagnosing higher-risk GBM or lower-risk GBM, wherein diagnostic methods practised on the human or animal body as well as methods for treatment of the human or animal body by surgery are excluded, and wherein said diagnosing is effected by the method of any one of claims 1 to 5.

8. The method of any one of claims 1 to 3, wherein said method comprises subjecting a sample taken from the GBM patient to an analytical method, said analytical method employing the means as defined in claim 7.

9. The method of any one of claims 1 to 5 or 8, the kit for use of claim 6, or the use of claim 7, wherein said higher-risk GBM is not responsive to postoperative radiotherapy.

10. The use of claim 7, wherein said kit and/or said means, respectively, further comprise(s) a carrier.

11. The use of any one of claims 7, 9 or 10, said kit further comprising a manual describing the method of any one of claims 1 to 5, 8 or 9.

12. The kit for use of any one of claims 6 or 9 to 11, or use of any one of the claims 7 or 9 to 11, wherein said probes and/or primers are immobilized on the carrier as defined in claim 10.

13. The kit for use of any one of claims 6 or 9 to 11, or use of any one of claims 7 or 9 to 11, wherein said stratifying and said diagnosing is to be effected for GBM patients, which are IDH1 mutation negative.

14. The method of any one of claims 1 to 5 or 9, wherein said determining comprises the use of probes and/or primers, which are specific for the respective miRNA, said use preferably being for determining the expression level of the respective miRNAs.

**Patentansprüche**

1. Verfahren zum Stratifizieren von Glioblastoma multiforme (GBM),
   wobei das Verfahren das Bestimmen aller vier Expressionsniveaus der miRNAs hsa-miR-615-5p, hsa-miR-125a-5p, hsa-let-7a-5p und hsa-let-7b-5p für einen GBM-Patienten umfasst oder daraus besteht,
   wobei

   (a) je mehr das Expressionsniveau von hsa-miR-615-5p erhöht ist und je mehr die Expressionsniveaus von hsa-miR-125a-5p, hsa-let-7a-5p und hsa-let-7b-5p verringert sind, desto schlechter ist die Prognose für den Patienten;
   (b) das Bestimmen, ob ein Expressionsniveau erhöht oder verringert ist, durch Vergleich mit einem Referenzzustand erfolgt, wobei der Referenzzustand die durchschnittlichen Expressionsniveaus von hsa-miR-615-5p, hsa-miR-125a-5p, hsa-let-7a-5p und hsa-let-7b-5p bei GBM entsprechend sind;
   und
   (c) die Diagnoseverfahren, die am menschlichen Körper oder Tierkörper durchgeführt werden, sowie chirurgische Behandlungsverfahren an dem menschlichen Körper oder Tierkörper ausgeschlossen sind.

2. Verfahren nach Anspruch 1, wobei

   (i) ein Expressionsniveau von hsa-miR-615-5p, das erhöht ist, und Expressionsniveaus von hsa-miR-125a-5p, hsa-let-7a-5p und hsa-let-7b-5p, die verringert sind, eine schlechte Prognose anzeigen; und
   (ii) ein Expressionsniveau von hsa-miR-615-5p, das verringert ist, und Expressionsniveaus von hsa-miR-125a-5p, hsa-let-7a-5p und hsa-let-7b-5p, die erhöht sind, eine gute Prognose anzeigen.

3. Verfahren nach Anspruch 1 oder 2, wobei das Verfahren nicht das Bestimmen der Expressionsniveaus von anderen miRNAs oder RNAs als allen vier von hsa-miR-615-5p, hsa-miR-125a-5p, hsa-let-7a-5p und hsa-let-7b-5p umfasst, wobei das Verfahren optional das Quantifizieren des (der) Expressionsniveau(s) von einem oder mehreren Standard-RNAs umfasst.

4. Verfahren zur Diagnose von GBM mit höherem Risiko oder GBM mit niedrigerem Risiko, wobei das Verfahren umfasst oder daraus besteht, eine von einem GMB-Patienten entnommene Probe einem Verfahren zum Bestimmen von Expressionsniveaus und zum Bestimmen eines Risikowertes R für den GMB-Patienten zu unterziehen, wobei R wie folgt definiert ist:

$$R = \Sigma_i\, a_i f_i$$

wobei sich

i von 1 bis 4 erstreckt;
$a_1$ zwischen 0,30 und 0,40 liegt und vorzugsweise 0,33 ist; $a_2$ zwischen -0,35 und -0,25 liegt und vorzugsweise -0,28 ist; $a_3$ zwischen 0,45 und 0,55 liegt und vorzugsweise 0,51 ist und $a_4$ zwischen -1,00 und -0,90 liegt und vorzugsweise -0,97 ist; und

$f_1$ bis $f_4$ die Expressionsniveaus der miRNAs hsa-miR-615-5p, hsa-miR-125a-5p, hsa-let-7a-5p und hsa-let-7b-5p sind;

wobei ein durchschnittlicher Risikowert $R_{av}$ als der Durchschnitt von R in dem GBM definiert ist;

wobei ein Wert von (R - $R_{av}$), der größer als Null ist, ein GBM mit höherem Risiko anzeigt, und ein Wert von (R - $R_{av}$), der kleiner als Null ist, ein GBM mit einem niedrigeren Risiko anzeigt; und

wobei die Diagnoseverfahren, die am menschlichen Körper oder Tierkörper durchgeführt werden, sowie chirurgische Behandlungsverfahren am menschlichen Körper oder Tierkörper ausgeschlossen sind.

5. Verfahren nach einem der Ansprüche 1 bis 4, wobei der GBM-Patient Isocitrat-Dehydrogenase 1 (IDH1)-Mutations-negativ ist.

6. Kit, bestehend aus Mitteln zur Detektion und/oder Quantifizierung aller vier der folgenden miRNAs: hsa-miR-615-5p, hsa-miR-125a-5p, hsa-let-7a-5p und hsa-let-7b-5p, wobei die Mittel aus Sonden oder Primern bestehen, die spezifisch für die jeweiligen miRNAs sind,
zur Verwendung in einem Verfahren zum Stratifizieren von GBM oder einem Verfahren zur Diagnose von GBM mit höherem Risiko oder GBM mit niedrigerem Risiko, wobei das Verfahren das Verfahren nach irgendeinem der Ansprüche 1 bis 5 ist.

7. Verwendung des Kits nach Anspruch 6 zum Stratifizieren von GBM oder zur Diagnose von GBM mit höherem Risiko oder GBM mit niedrigerem Risiko, wobei Diagnoseverfahren, die am menschlichen Körper oder Tierkörper durchgeführt werden, sowie chirurgische Behandlungsverfahren am menschlichen Körper oder Tierkörper ausgeschlossen sind, und wobei die Diagnose durch das Verfahren nach irgendeinem der Ansprüche 1 bis 5 durchgeführt wird.

8. Verfahren nach einem der Ansprüche 1 bis 3, wobei das Verfahren umfasst, eine dem GBM-Patienten entnommene Probe einem analytischen Verfahren zu unterziehen, wobei das analytische Verfahren die Mittel wie definiert in Anspruch 7 anwendet.

9. Verfahren nach irgendeinem der Ansprüche 1 bis 5 oder 8, Kit zur Verwendung nach Anspruch 6 oder Verwendung nach Anspruch 7, wobei das GBM mit höherem Risiko nicht auf eine postoperative Strahlentherapie anspricht.

10. Verwendung nach Anspruch 7, wobei das Kit und/oder die Mittel, entsprechend, ferner einen Träger umfasst/ umfassen.

11. Verwendung nach irgendeinem der Ansprüche 7, 9 oder 10, wobei das Kit ferner eine Anleitung umfasst, die das Verfahren nach irgendeinem der Ansprüche 1 bis 5, 8 oder 9 beschreibt.

12. Kit zur Verwendung nach irgendeinem der Ansprüche 6 oder 9 bis 11 oder Verwendung nach irgendeinem der Ansprüche 7 oder 9 bis 11, wobei die Sonden und/oder Primer auf dem Träger wie definiert in Anspruch 10 immobilisiert sind.

13. Kit zur Verwendung nach irgendeinem der Ansprüche 6 oder 9 bis 11 oder Verwendung nach irgendeinem der Ansprüche 7 oder 9 bis 11, wobei das Stratifizieren und die Diagnose für GBM-Patienten durchgeführt wird, die IDH1-Mutations-negativ sind.

14. Verfahren nach irgendeinem der Ansprüche 1 bis 5 oder 9, wobei das Bestimmen die Verwendung von Sonden und/oder Primern umfasst, die für die jeweilige miRNA spezifisch sind, wobei die Verwendung bevorzugt der Bestimmung des Expressionsniveaus der jeweiligen miRNAs dient.

## Revendications

1. Procédé de stratification du glioblastome multiforme (GBM),
ledit procédé comprenant ou consistant à déterminer pour un sujet atteint de GBM tous les quatre des niveaux d'expression de miARNs hsa-miR-615-5p, hsa-miR-125a-5p, hsa-let-7a-5p et hsa-let-7b-5p dans lequel

(a) plus le niveau d'expression de hsa-miR-615-5p est élevé, et plus les niveaux d'expression de hsa-miR-125a-5p, hsa-let-7a-5p et hsa-let-7b-5p sont diminués, plus le pronostic est mauvais pour ledit sujet;

(b) la détermination si un niveau d'expression est élevé ou diminué se fait par comparaison avec un état de référence, ledit état de référence étant les niveaux d'expression moyens en GBM de hsa-miR-615-5p, hsa-miR-125a-5p, hsa-let-7a-5p et hsa-let-7b-5p respectivement; et

(c) des procédés de diagnostic pratiqués sur le corps humain ou animal ainsi que des procédés de traitement du corps humain ou animal par la chirurgie sont exclus.

2. Procédé selon la revendication 1, dans lequel

(i) un niveau d'expression de hsa-miR-615-5p, qui est élevé, et des niveaux d'expression de hsa-miR-125a-5p, hsa-let-7a-5p et hsa-let-7b-5p, qui sont diminués, indiquent un mauvais pronostic; et
(ii) un niveau d'expression de hsa-miR-615-5p, qui est diminué, et des niveaux d'expression de hsa-miR-125a-5p, hsa-let-7a-5p et hsa-let-7b-5p, qui sont élevées, indiquent un bon pronostic.

3. Procédé selon la revendication 1 ou 2, dans lequel ledit procédé ne comprend pas l'évaluation des niveaux d'expression de miARNs ou ARNs autre que tous les quatre de hsa-miR-615-5p, hsa-miR-125a-5p, hsa-let-7a-5p et hsa-let-7b-5p, dans lequel ledit procédé comporte facultativement la quantification des niveaux d'expression d'un ou plusieurs ARNs standards.

4. Procédé de diagnostic d'un GBM à risque plus élevé ou d'un GBM à risque plus faible, ledit procédé comportant ou consistant à soumettre un échantillon prélevé sur un sujet atteint de GBM à un procédé permettant de déterminer des niveaux d'expression et déterminer un score de risque R pour le sujet atteint de GBM, R étant défini comme suit:

$$R = \Sigma_i\, a_i f_i$$

où

i va de 1 à 4;
$a_1$ est compris entre 0,30 et 0,40 et est de préférence 0,33 ist; $a_2$ est compris entre -0,35 et -0,25 et est de préférence -0,28; $a_3$ est compris entre 0,45 et 0,55 et est de préférence 0,51 et $a_4$ est compris entre -1,00 et -0,90 et est de préférence -0,97; et
$f_1$ bis $f_4$ sont les niveaux d'expression des miARNs hsa-miR-615-5p, hsa-miR-125a-5p, hsa- let-7a-5p et hsa-let-7b-5p;
dans lequel un score de risque moyen $R_{av}$ est défini comme la moyenne de R en GBM;
dans lequel une valeur de ($R - R_{av}$), qui est supérieure à zéro, indique un risque plus élevé de GBM, et une valeur de ($R - R_{av}$), qui est inférieure à zéro, indique un risque plus faible de GBM; et
dans lequel des procédés de diagnostic pratiqués sur le corps humain ou animal ainsi que des procédés de traitement du corps humain ou animal par la chirurgie sont exclus.

5. Procédé selon l'une quelconque des revendications 1 à 4, dans lequel ledit sujet atteint de GBM est négatif concernant la mutation de l'isocitrate déshydrogénase 1 (IDH1).

6. Kit comportant des moyens pour la détection et/ou la quantification de tous les quatre des miARNs suivants: hsa-miR-615-5p, hsa-miR-125a-5p, hsa-let-7a-5p et hsa-let-7b-5p, dans lequel lesdits moyens comportent des sondes et amorces, qui sont spécifiques pour le miARNs respectifs, à utiliser dans un procédé de stratification du GBM ou un procédé de diagnostic d'un GBM à risque plus élevé ou d'un GBM à risque plus faible, ledit procédé étant le procédé selon l'une quelconque des revendications 1 à 5.

7. Utilisation du kit selon la revendication 6 pour la stratification du GBM ou pour le diagnostic d'un GBM à risque plus élevé ou d'un GBM à risque plus faible, dans lequel des procédés de diagnostic pratiqués sur le corps humain ou animal ainsi que des procédés de traitement du corps humain ou animal par la chirurgie sont exclus, et dans lequel ledit diagnostic est effectué par le procédé selon l'une quelconque des revendications 1 à 5.

8. Procédé selon l'une quelconque des revendications 1 à 3, dans lequel ledit procédé consiste à soumettre un échantillon prélevé sur un sujet atteint de GBM à un procédé analytique, ledit procédé analytique utilisant les moyens selon la revendication 7.

9. Procédé selon l'une quelconque des revendications 1 à 5 ou 8, kit à utiliser selon la revendication 6 ou utilisation selon la revendication 7, dans lequel ledit GBM à risque plus élevé ne répond pas à une radiothérapie postopératoire.

10. Utilisation selon la revendication 7, dans lequel ledit kit et/ou lesdits moyens respectivement comporte(nt) en outre un support.

11. Utilisation selon l'une quelconque des revendications 7, 9 ou 10, dans lequel le kit comporte en outre un mode d'emploi décrivant le procédé selon l'une quelconque des revendications 1 à 5, 8 ou 9.

12. Kit à utiliser selon l'une quelconque des revendications 6 ou 9 à 11 ou utilisation selon l'une quelconque des revendications 7 ou 9 à 11, dans lequel lesdites sondes et/ou amorces sont immobilisées sur le support selon la revendication 10.

13. Kit à utiliser selon l'une quelconque des revendications 6 ou 9 à 11 ou utilisation selon l'une quelconque des revendications 7 ou 9 à 11, dans lequel ladite stratification et ledit diagnostic doivent être effectués pour les sujets atteint de GBM qui sont négatifs concernant la mutation IDH1.

14. Procédé selon l'une quelconque des revendications 1 à 5 ou 9, dans lequel ladite détermination comporte l'utilisation des sondes et/ou amorces, qui spécifiques pour le miARN respectif, ladite utilisation étant de préférence destinée à déterminer le niveau d'expression des miARNs respectifs.

**Figure 1**

**A**

**Figure 1 continued**

B

**Figure 1 continued**

C

lower-risk higher-risk
p=0.034

p=0.164

**Figure 2**

A

**Age-distributions of cohorts**

Figure 2 continued

B

**Figure 2 continued**

C

# Overall survival miRNA signature validation cohort

**Figure 2 continued**

D

**Figure 3**

All – Overall survival

n(Low-risk)=22
n(High-risk)=19
P=0.123136
Hazard ratio=1.72 (0.86–3.48)

Cumulative proportion surviving

Low-risk (med. surv. 726 days
High-risk (med. surv. 498 days)

Survival time [years]

**Figure 4**

IDH1-negative – Overall survival

n(Low−risk)=20
n(High−risk)=16
P=0.035792
Hazard ratio=2.15 (1.04−4.44)

Low−risk (med. surv. 726 days
High−risk (med. surv. 367 days)

Cumulative proportion surviving

Survival time [years]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012089753 A
- EP 15202328 PCT

### Non-patent literature cited in the description

- **FISHER et al.** *Neurol. Clin,* 2007, vol. 25, 867-90
- **LOUIS et al.** *Acta Neuropathol,* 2007, vol. 114, 97-109
- **STUPP et al.** *Lancet. Oncol.,* 2009, vol. 10, 459-66
- **STUPP et al.** *N. Engl. J. Med.,* 2005, vol. 352, 987-96
- **CHINOT et al.** *N. Engl. J. Med.,* 2014, vol. 370 (8), 709-722
- **GILBERT et al.** *N. Engl. J. Med.,* 2014, vol. 370, 699-708
- **STUPP et al.** *Lancet. Oncol.,* 2014, vol. 15, 1100-8
- **NIYAZ et al.** *Radiat Oncol,* 2014, vol. 9, 299
- **NIYAZI et al.** *Radiat Oncol,* 2013, vol. 8, 287
- **NIYAZI et al.** *Radiat. Oncol.,* 2011, vol. 6, 177
- **NIYAZI et al.** *Radiat. Oncol.,* 2014, vol. 9, 299
- **WEBER et al.** *Radiother Oncol,* 2009, vol. 93, 586-92
- **CYRAN et al.** *Radiat. Oncol.,* 2014, vol. 9, 3
- **GILBERT et al.** *RTOG 0625: A phase II study of bevacizumab with irinotecan in recurrent glioblastoma (GBM),* 2009
- **NIYAZI et al.** *Radiother Oncol,* 2011, vol. 98, 1-14
- **STALL et al.** *Radiat. Oncol.,* 2010, vol. 5, 5
- **BEAL et al.** *Radiat. Oncol.,* 2011, vol. 6, 2
- **FLIEGER et al.** *J. Neurooncol.,* 2014, vol. 117, 337-45
- **NIYAZI et al.** *Int. J. Radiat. Oncol. Biol. Phys.,* 2010
- **STUPP et al.** *J. Clin. Oncol.,* 2010, vol. 28, 2712-8
- **WELSH et al.** *Radiat Oncol,* 2009, vol. 4, 69
- **FRENEL et al.** *Bulletin Du Cancer,* 2009, vol. 96, 357-367
- **ADEBERG et al.** *Radiation Oncology (London, England),* 2014, vol. 9, 95
- **HEGI et al.** *J. Clin. Oncol.,* 2008, vol. 26, 4189-99
- **KIM HAK JAE et al.** *Radiation oncology (London, England),* 2012, vol. 7, 39
- **REIFENBERGER et al.** *Int. J. Cancer.,* 2014, vol. 135, 1822-31
- **SRINIVASAN et al.** A ten-microRNA expression signature predicts survival in glioblastoma. *PLoS ONE,* 2011, vol. 6 (3
- **HA MINJU ; KIM V. NARRY.** *Nature Reviews Molecular Cell Biology,* 2014, vol. 15, 509-524
- **CHENG WEN et al.** *Oncotarget,* 2015
- **DE SMAELE et al.** *Brain Res,* 2010, vol. 1338, 100-11
- **GAO et al.** *Oncogene,* 2014, vol. 0
- **JIANG et al.** *American Journal of Pathology,* 2010, vol. 177, 29-38
- **MANTEROLA et al.** *Neuro-Oncology,* 2014
- **SANA et al.** *Carcinogenesis,* 2014, vol. 35, 2756-2762
- **SHOU et al.** *Experimental and Therapeutic Medicine,* 2015, vol. 9, 167-171
- **ZHANG et al.** *PLoS ONE,* 2014, vol. 9, e96908
- **GRIFFITHS-JONES S ; GROCOCK RJ ; VAN DONGEN S ; BATEMAN A ; ENRIGHT AJ.** *Nucl. Acids Res,* 2006, vol. 34, D140-D144
- **REUSS et al.** *Acta Neuropathol,* 2015, vol. 129, 133-146
- Cancer Genome Atlas Research Network. Comprehensive genomic characterization defines human glioblastoma genes and core pathways. *Nature,* 2008, vol. 455, 1061-8
- **STUPP et al.** *Lancet Oncol,* 2009, vol. 10, 459-466
- **NIYAZI et al.** *Oncotarget,* 2016, vol. 7, 45764-45775
- **CAI et al.** *Oncoscience,* 2016, vol. 3, 258-265